# EUROPEAN PATENT APPLICATION

(11) **EP 4 567 126 A1**
(43) Date of publication of application: **11.06.2025**
(21) Application number: 23850065.6
(22) Date of filing: 01.08.2023
(51) Int. Cl.: C12Q 1/6813, C12M 1/34, C12Q 1/6876, C12N 15/09

(54) **EVALUATION METHOD, COMPLEX, AND KIT**

(30) Priority: 01.08.2022 JP 2022122819
(71) Applicant: Toppan Holdings Inc., Tokyo 110-0016 (JP)
(72) Inventor: KUBO Yuji, Tokyo 110-0016 (JP); MAKINO Yoichi, Tokyo 110-0016 (JP)
(74) Representative: Plasseraud IP
(86) International application number: PCT/JP2023/028056
(87) International publication number: WO 2024/029511

(57) **Abstract**

The disclosure provides an evaluation method that is a method of evaluating whether a protein of interest binds to a target protein, including a step (a) of bringing a protein of interest, a target protein, a first specific binding substance which is for the protein of interest and is labeled with a first single-stranded nucleic acid fragment, and a second specific binding substance which is for the target protein and is labeled with a second single-stranded nucleic acid fragment, into contact with each other in a container, and forming as a result a complex comprising the protein of interest, the target protein, and the first and second specific binding substances when the protein of interest binds to the target protein, with a double-stranded nucleic acid being formed by hybridization of the first single-stranded nucleic acid fragment with the second single-stranded nucleic acid fragment; and a step (b) of detecting formation of the double-stranded nucleic acid, wherein detection of formation of the double-stranded nucleic acid indicates binding of the protein of interest to the target protein.

## Description

### [Technical Field]

The present invention relates to methods of evaluating mutant protein activity. More specifically, the present invention relates to methods of evaluating whether a protein of interest binds to a target protein, complexes, kits for evaluating whether a protein of interest binds to a target protein, and methods of evaluating whether a protein of interest phosphorylates a target protein.

The present application claims priority to Japanese Patent Application No. 2022-122819 filed August 1, 2022, the contents of which are incorporated herein by reference.

### [Background Art]

With the advent of comprehensive genetic analysis technology represented by next-generation sequencers, many genetic alterations and mechanisms of disease onset are becoming clearer. At the same time, numerous variants of unknown significance (hereinafter referred to as VUS) have been identified.

In current genomic medicine, the diagnostic rate and drug accessibility through genetic analysis are low. One of the reasons for this is the large number of VUS cases reported, and there is an urgent need to determine the significance of VUS and evaluate the VUS.

To determine the significance of VUS and evaluate the VUS, various scores, including Combined Annotation Dependent Depletion (https://cadd.gs.washington.edu/) score and MutPred2 (https://mutpred.mutdb.org/) score, are calculated and can be referenced. However, computational predictions cannot be used as the sole evidence for evaluating pathogenicity because they may not match the actual phenotype.

One established method of experimentally evaluating VUS involves creating a plasmid with a mutation in the target gene, introducing the plasmid into cells to synthesize a protein, phosphorylating the protein synthesized in the cells, and then detecting the cell extract containing the phosphorylated protein using Western blotting or the like to qualitatively evaluate the VUS. However, such an evaluation method requires a lot of time, effort and cost.

Conventionally, protein quantitative detection has been performed using enzyme-linked immunosorbent assays (also referred to as ELISAs), etc., and nucleic acid quantification has been performed using real-time PCR methods etc.

As a method of detecting a target substance with higher accuracy, techniques for causing enzymatic reactions in a large number of microcompartments are being considered. These techniques are called digital measurements. Digital measurements include digital ELISAs and digital PCRs.

In digital measurements, a sample solution is divided into an extremely large number of micro-solutions. Then, signals from the individual micro-solutions are binarized and only whether the target substance is present is determined to measure the number of molecules of the target substance. Digital measurements can significantly improve detection sensitivity and quantifiability compared to ELISAs, real-time PCRs, etc. of the conventional art.

For example, Non-Patent Literature (NPTL) 1 describes a microwell array with microwells and channels for supplying reagents, etc., and describes performing a digital ELISA using the microwell array.

PTL 1 describes a proximity ligation assay (also referred to as PLA) which is a method of detecting a protein using an antibody modified with an oligonucleotide. This method utilizes a PCR method or RCA method for the detection.

PTL 2 describes a method of detecting protein interactions between two molecules using an antibody modified with an oligonucleotide. This method also utilizes a PCR method or RCA method for the detection.

Non-Patent Literature (NPTL) 3 describes a microwell array with microwells and channels for supplying reagents, etc., and describes expressing signal transduction using cells in the microwell array to detect phosphorylated proteins. The state of signal transduction is monitored by detecting phosphorylated proteins.

Alpha SureFire (PerkinElmer: https://www.perkinelmer.cojp/assays/tabid/346/Default.aspx) is an assay system with which kinase activity can be detected on a cell-by-cell basis without the need of washing procedures. In this system, phosphorylated proteins are detected from cell extracts.

### [Citation List]

### [Patent Literature]

PTL 1: JP2020-122799A
PTL 2: US10465235B

### [Non-Patent Literature]

NPTL 1: Kan C. W., et al., Isolation and detection of single molecules on paramagnetic beads using sequential fluid flows in microfabricated polymer array assemblies, Lab on a Chip, 12 (5), 977-985, 2012.
NPTL 2: Mohammed H., et al., Approaches for Assessing and Discovering Protein Interactions in Cancer., Mol Cancer Res, 11 (11), 1295-1302, 2013.
NPTL 3: Blazek M., et al., Proximity Ligation Assay for High-content Profiling of Cell Signaling Pathways on a Microfluidic Chip., Molecular & Cellular Proteomics 12: 10.1074/mcp.M113.032821, 3898-3907, 2013.

### [Summary of Invention]

### [Technical Problem]

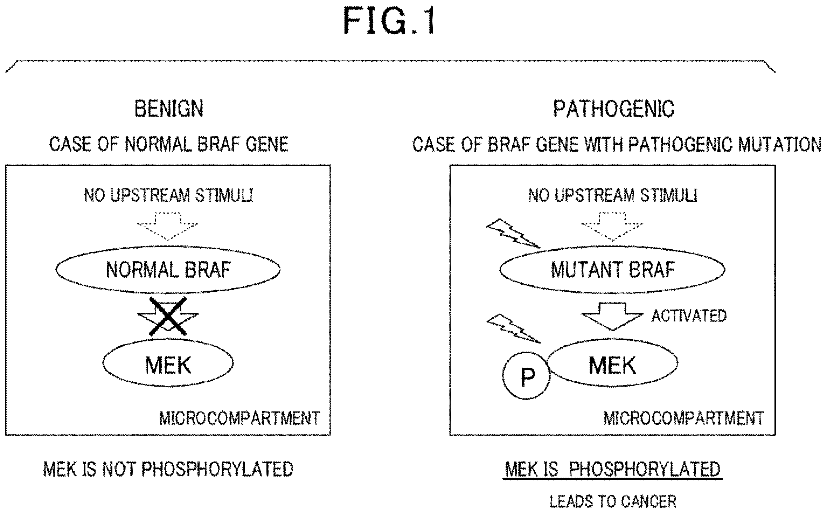

Living organisms have mechanisms for transmitting external stimuli into cells. In these mechanisms, various proteins temporarily bind to each other and transmit information. This information transmission system, which is referred to as a signal transduction system, is assumed by various protein molecules that mediate stimuli. Fig. 1 shows, as an example, part of signal transduction of mitogen-activated protein kinase (also referred to as MAPK).

Fig. 1 shows, as an example, the case of the mutant protein being BRAF, i.e., shows the case of the variants of unknown significance (i.e., VUS) in the BRAF gene being benign, and the case of the VUS therein being pathogenic. When the VUS is benign, MEK, the substrate of BRAF, is not phosphorylated unless there are upstream stimuli, as shown in Fig. 1 left. In contrast, when the VUS is pathogenic, BRAF is constantly activated and phosphorylates its substrate, MEK, even in the absence of upstream stimuli, as shown in Fig. 1 right. Such BRAF causes cancer.

The present invention aims to provide a technique for evaluating mutant protein activity easily and in a short period of time.

### [Solution to Problems]

The present invention includes the following aspects.
[1] A method of evaluating whether a protein of interest binds to a target protein, including a step (a) of bringing the protein of interest, the target protein, a first specific binding substance which is for the protein of interest and is labeled with a first single-stranded nucleic acid fragment, and a second specific binding substance which is for the target protein and is labeled with a second single-stranded nucleic acid fragment, into contact with each other in a container, and forming as a result a complex comprising the protein of interest, the target protein, the first specific binding substance, and the second specific binding substance when the protein of interest binds to the target protein, with a double-stranded nucleic acid being formed by hybridization of at least part of the first single-stranded nucleic acid fragment with at least part of the second single-stranded nucleic acid fragment; and a step (b) of detecting formation of the double-stranded nucleic acid, wherein detection of formation of the double-stranded nucleic acid indicates binding of the protein of interest to the target protein.
[2] The method according to [1], wherein the step (a) includes a step (a1) of synthesizing the protein of interest in the container using a cell-free protein synthesis system; and a step (a2) of bringing the protein of interest, the target protein, the first specific binding substance, and the second specific binding substance, into contact with each other in the container, and forming as a result a complex comprising the protein of interest, the target protein, the first specific binding substance, and the second specific binding substance when the protein of interest binds to the target protein, with a double-stranded nucleic acid being formed by hybridization of at least part of the first single-stranded nucleic acid fragment with at least part of the second single-stranded nucleic acid fragment.
[3] The method according to [2], wherein in the step (a2), adenosine triphosphate (ATP) is additionally brought into contact with the protein of interest, the target protein, the first specific binding substance, and the second specific binding substance.
[4] The method according to any of [1] to [3], wherein the method does not include any cleaning process.
[5] The method according to any of [1] to [4], wherein the container comprises wells, and each of the wells has a volume of 10 fL to 100 pL.
[6] The method according to any of [1] to [5], wherein the first single-stranded nucleic acid fragment and the second single-stranded nucleic acid fragment each have a length of 10 to 200 bases.
[7] The method according to any of [1] to [6], wherein the step of detecting formation of the double-stranded nucleic acid is performed through an invasive cleavage assay.
[8] A complex comprising a protein of interest, a target protein, a first specific binding substance which is for the protein of interest and is labeled with a first single-stranded nucleic acid fragment, and a second specific binding substance which is for the target protein and is labeled with a second single-stranded nucleic acid fragment, with a double-stranded nucleic acid being formed by hybridization of at least part of the first single-stranded nucleic acid fragment with at least part of the second single-stranded nucleic acid fragment.
[9] A kit for evaluating whether a protein of interest binds to a target protein, including a well array having a plurality of wells; a first specific binding substance which is for the protein of interest and is labeled with a first single-stranded nucleic acid fragment; and a second specific binding substance which is for the target protein and is labeled with a second single-stranded nucleic acid fragment.
[10] The kit according to [9], further including ATP.
[11] The kit according to [9] or [10], further including a sealing liquid for sealing openings of the wells.
[12] The kit according to any one of [9] to [11], further including a reagent for detecting a double-stranded nucleic acid formed by hybridization of at least part of the first single-stranded nucleic acid fragment with at least part of the second single-stranded nucleic acid fragment.
[13] A method of evaluating whether a protein of interest phosphorylates a target protein, including a step (a') of bringing the protein of interest, the target protein, the second specific binding substance which is for the target protein and is labeled with a second single-stranded nucleic acid fragment, a third specific binding substance which is for the target protein undergone phosphorylation and is labeled with a third single-stranded nucleic acid fragment, and ATP, into contact with each other in a container, and forming as a result a complex comprising the target protein, the second specific binding substance, and the third specific binding substance when the target protein is phosphorylated, with a double-stranded nucleic acid being formed by hybridization of at least part of the second single-stranded nucleic acid fragment with at least part of the third single-stranded nucleic acid fragment; and a step (b) of detecting formation of the double-stranded nucleic acid, wherein detection of formation of the double-stranded nucleic acid indicates phosphorylation of the target protein.

### [Advantageous Effects of the Invention]

According to the present invention, there can be provided a technique for evaluating mutant protein activity easily and in a short period of time. According to the present invention, for example, a functional analysis (evaluation) of VUS can be performed easily, efficiently, and in a short period of time.

### [Brief Description of Drawings]

Fig. 1 is a set of diagrams illustrating a benign mutation and a pathogenic mutation.
Fig. 2 is a schematic diagram illustrating a method of evaluating whether a protein of interest binds to a target protein.
Fig. 3 is a schematic cross-sectional view illustrating an example of a fluidic device.
Fig. 4 is a schematic cross-sectional view illustrating a method of evaluating whether a protein of interest binds to a target protein.
Fig. 5 is a schematic cross-sectional view illustrating a method of evaluating whether a protein of interest binds to a target protein.
Fig. 6 is a schematic cross-sectional view illustrating an example of a fluidic device.
Fig. 7 is a schematic cross-sectional view illustrating a method of evaluating whether a protein of interest binds to a target protein.
Fig. 8 is a schematic cross-sectional view illustrating a method of evaluating whether a protein of interest binds to a target protein.
Fig. 9 is a schematic diagram illustrating an example of an invasive cleavage assay (ICA) method.
Fig. 10 is a schematic diagram illustrating a method of evaluating the activity of a protein of interest.
Fig. 11 is a schematic diagram illustrating a method of evaluating whether a protein of interest phosphorylates a target protein.
Fig. 12 is a graph showing the results of Experimental Example 1.
Fig. 13 is a graph showing the results of Experimental Example 1.
Fig. 14 is a set of graphs showing the results of Experimental Example 1.
Fig. 15 is a set of graphs showing the results of Experimental Example 2.
Fig. 16 is a set of graphs showing the results of Experimental Example 3.
Fig. 17 is a graph showing the results of Pattern 1 of Experimental Example 4.
Fig. 18 is a graph showing the results of Pattern 2 of Experimental Example 4.
19 is a histogram showing measurements of fluorescence intensity over time in immuno-ICA reactions of Experimental Example 5.

### [Description of Embodiments]

With reference to the drawings where necessary, embodiments of the present invention will be described in detail. Throughout the drawings, the same or corresponding parts are denoted by the same or corresponding reference signs to omit repeated explanation. The dimension ratios in some drawings are exaggerated for convenience of explanation and are not necessarily to scale.

### [Method of evaluating whether protein of interest binds to target protein]

In an embodiment, the present invention provides a method of evaluating whether a protein of interest binds to a target protein, the method including a step (a) of bringing the protein of interest, the target protein, a first specific binding substance which is for the protein of interest and is labeled with a first single-stranded nucleic acid fragment, and a second specific binding substance which is for the target protein and is labeled with a second single-stranded nucleic acid fragment, into contact with each other in a container, and forming as a result a complex comprising the protein of interest, the target protein, the first specific binding substance, and the second specific binding substance when the protein of interest binds to the target protein, with a double-stranded nucleic acid being formed by hybridization of at least part of the first single-stranded nucleic acid fragment with at least part of the second single-stranded nucleic acid fragment; and a step (b) of detecting formation of the double-stranded nucleic acid, wherein detection of formation of the double-stranded nucleic acid indicates binding of the protein of interest to the target protein.

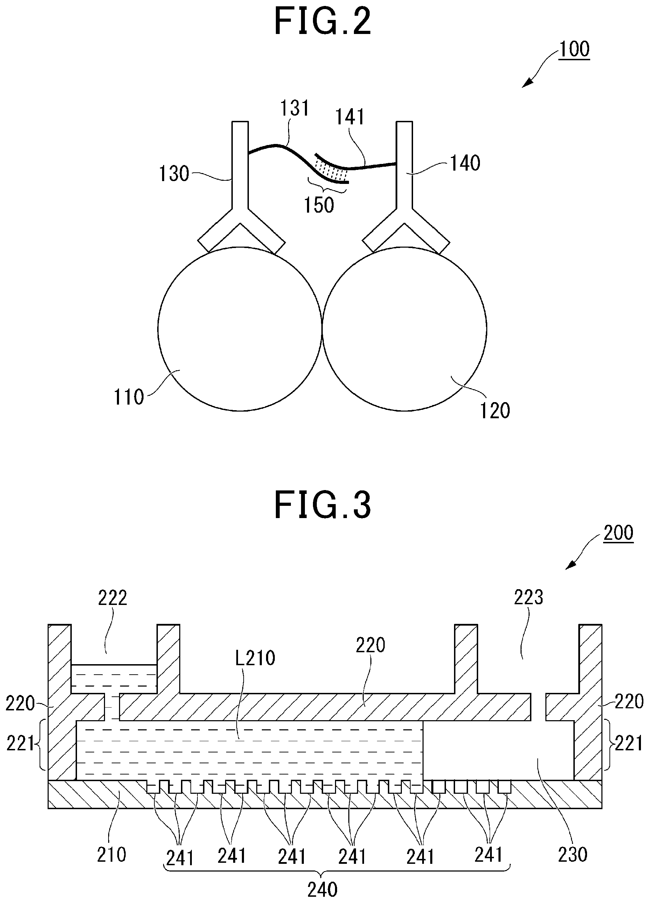

Fig. 2 is a schematic diagram illustrating a method according to the present embodiment. As shown in Fig. 2, in the method of the present embodiment, in step (a) first, a protein of interest 110, a target protein 120, a first specific binding substance 130 which is for the protein of interest 110 and is labeled with a first single-stranded nucleic acid fragment 131, and a second specific binding substance 140 which is for the target protein 120 and is labeled with a second single-stranded nucleic acid fragment 141 are brought into contact with each other in a container.

As a consequence, if the protein of interest 110 binds to the target protein 120, a complex is formed comprising the protein of interest 110, the target protein 120, the first specific binding substance 130, and the second specific binding substance 140, with a double-stranded nucleic acid (also referred to as double-stranded nucleic acid region) 150 formed by hybridization of at least part of the first single-stranded nucleic acid fragment 131 with at least part of the second single-stranded nucleic acid fragment 141.

Subsequently, in step (b), formation of the double-stranded nucleic acid 150 is detected. If formation of the double-stranded nucleic acid 150 is detected, it indicates that the protein of interest 110 binds to the target protein 120 (i.e., they interact each other).

The method according to the present embodiment may be applied to the case where the protein of interest 110 does not bind to the target protein 120 (i.e., they do not interact each other). In that case, formation of the double-stranded nucleic acid 150 is not detected. Detecting formation of the double-stranded nucleic acid 150 will be described later.

As will be described later in examples, according to the present invention, mutant protein activity can be evaluated easily and in a short period of time. For example, a functional analysis (i.e., evaluation) of VUS can be performed easily, efficiently, and in a short period of time.

From the perspective that at least part of the first single-stranded nucleic acid fragment 131 is required to be able to hybridize with at least part of the second single-stranded nucleic acid fragment 141, the first single-stranded nucleic acid fragment 131 may have a length of 10 to 200 bases. Similarly, the second single-stranded nucleic acid fragment 141 may also have a length of 10 200 bases. The double-stranded nucleic acid 150 formed by hybridization of at least part of the first single-stranded nucleic acid fragment 121 with at least part of the second single-stranded nucleic acid fragment 131 is preferred to have a length of approximately 7 to 30 bases, e.g., may be 9, 12 or 15 bases.

In the method according to the present embodiment, the protein of interest 110 may be a protein having variants of unknown significance (i.e., VUS). The protein of interest 110 may be, for example, a kinase. The protein of interest may be, more specifically, proteins in intracellular signal transduction pathways, including, for example, BRAF, A-RAF, Raf, MAP3K 4/12, MAP3K11, ASK1, and TAK1. In this case, whether the protein having VUS is in a constantly activated state can be evaluated based on whether the protein of interest binds to the target protein.

### (Container)

The method of the present embodiment can be performed using digital measurement. When performing the method of the present embodiment using digital measurement, the container is preferred to comprise wells, and the wells are preferred to constitute a well array with the multiple wells arranged therein. The well array is preferred to be disposed in a channel of a fluidic device.

### (Fluidic device)

Fig. 3 is a schematic cross-sectional view illustrating an example of a fluidic device with which the method of the present embodiment can be preferably performed. As shown in Fig. 3, a fluidic device 200 includes a substrate 210 and a lid member 220 disposed facing the substrate 210. The lid member 220 includes a protrusion 221. The protrusion 221 has an end contacting the substrate 210. The fluidic device 200 includes a well array 240 which is formed on one surface of the substrate 210, being integrated therewith, and faces the lid member 220. The well array 240 includes a plurality of wells 241. The lid member 220 may be welded or adhered to the substrate 210.

The wells 241 are open at the surface of the substrate 210. The shape, size, and arrangement of the wells 241 are not specifically limited; however, the wells 241 are preferred to be microwells with small volumes. For example, each well 241 may have a volume of approximately 10 fL to 100 pL. In the fluidic device 200, the multiple wells 241 with the same shape and size configure the well array 240. The same shape and size may refer to the same shape and volume required for the digital measurement, accepting a manufacturing error variation.

Each well 241 may have a diameter of, for example, approximately 1 µm to 10 µm. Each well 241 may have a depth of, for example, approximately 1 µm to 10 µm. The arrangement of the wells 241 is not specifically limited, and may be arranged, for example, in a triangular lattice shape or a square lattice shape, or may be randomly arranged.

In the fluidic device 200, there is a space formed between the well array 240 and the lid member 220 due to the presence of the protrusion 221. The space configures a channel 230. The channel 230 functions as a path for supplying a liquid in which a protein of interest, target protein, first specific binding substance, second specific binding substance, etc. are dispersed, and for supplying a sealing liquid described later. The shape, structure, volume, etc. of the channel 230 are not specifically limited, but the height of the channel 230 (i.e., the distance between the surface of the substrate 210 and the surface of the lid member 220 facing the substrate 210) is preferred to be, for example, 500 µm of less, more preferred to be, for example, 300 µm or less, even more preferred to be, for example, 200 µm or less, and most preferred to be, for example, 100 µm or less.

The protrusion 221 may be formed integrally with the lid member 220. For example, the lid member 220 can be formed by molding a thermoplastic resin fluid into a plate shape with a protrusion 221 using a mold. The lid member 220 may be provided with an inlet port 222 and an outlet port 223 for reagents.

If the lid member 220 has a protrusion 221, the lid member 220 and the substrate 210 are overlapped with each other so that the protrusion 221 is brought into contact with the surface of the substrate 210 in which the wells 241 are open. As a consequence, the space between the lid member 220 and the substrate 210 serves as a channel 230. The lid member 220 and the substrate 210 may be welded together by laser welding or the like.

### (Fluidic device modification 1)

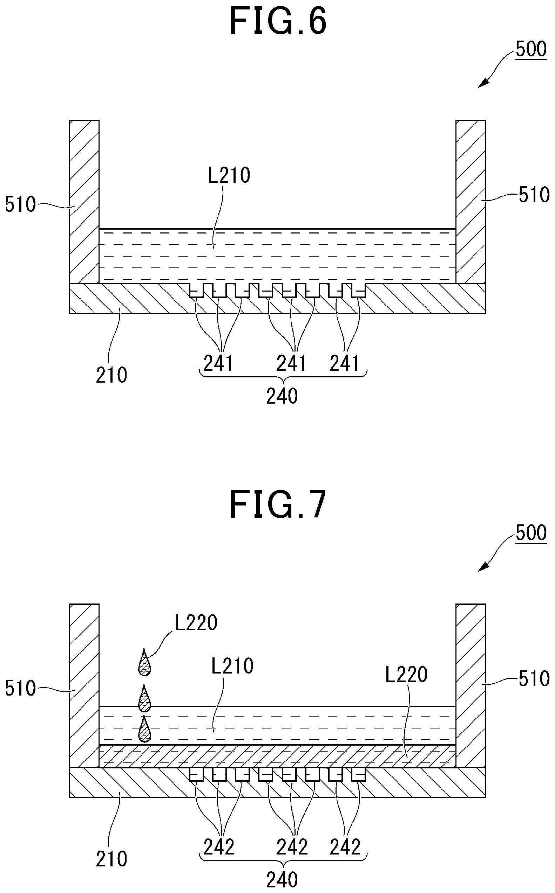

The fluidic device used for the method of the present embodiment is not limited to the fluidic device 200 described above. Fig. 6 is a schematic cross-sectional view illustrating an example of a fluidic device. As shown in Fig. 6, a fluidic device 500 includes a substrate 210 and a wall member 510. The fluidic device 500 includes a well array 240 formed on one surface of the substrate 210, being integrated therewith. The well array 240 includes a plurality of wells 241. The fluidic device 500 differs mainly from the fluidic device 200 described above in that it does not include a lid member 220.

### (Fluidic device modification 2)

In the fluidic device 200 described above, the lid member 220 is formed integrally with the protrusion 221. However, the lid member 220 and the protrusion 221 may be molded as separate bodies.

The fluidic devices 200 and 500 described above each include a well array 240 formed on one surface of the substrate 210, being integrated therewith. However, the well array does not have to be formed integrally with the substrate 210. For example, a well array 240 molded separately from the fluidic device may be disposed on the substrate 210 of the fluidic device. Alternatively, a resin layer may be deposited on the surface of the substrate 210, and a well array may be formed in the resin layer by etching or the like.

### (Materials of fluidic device)

The substrate 210 may be made of a resin, for example. The type of the resin is not specifically limited, but it is preferred to be a resin resistant to reagents and sealing liquids. If the signal to be detected is fluorescence, the resin is preferred to have low autofluorescence. Examples of the resin having low autofluorescence include cycloolefin polymers, cycloolefin copolymers, silicones, polypropylenes, polycarbonates, polystyrenes, polyethylenes, polyvinyl acetates, fluororesins, and amorphous fluororesins, but are not limited thereto.

The substrate 210 may be provided with a plurality of wells 241 formed on one surface thereof in the thickness direction. Examples of the method of forming wells in a resin include injection molding, thermal imprinting, and optical imprinting.

Alternatively, for example, a fluororesin may be deposited on the substrate 210 and processed by etching or the like to form a well array. The fluororesin used may be, for example, CYTOP (trademark) (manufactured by AGC Inc.) or the like.

If the fluidic device includes a lid member 220, the material of the lid member 220 is preferred to be a resin with low autofluorescence, examples of which include thermoplastic resins such as cycloolefin polymers and cycloolefin copolymers.

The lid member 220 may be formed of a material that does not transmit light with a wavelength near the wavelength detected in fluorescence observation for signals, or a material that is completely opaque to light. For example, the lid member 220 may be made of a thermoplastic resin with an addition of carbon, metal particles, etc.

### (Method of the present embodiment)

Referring to Figs. 3 to 5 where necessary, the method of the present embodiment will be described, taking an example of using the fluidic device 200. The method of the present embodiment is a method of evaluating whether a protein of interest binds to a target protein, and includes a step (a) of bringing the protein of interest 110, the target protein 120, the first specific binding substance 130 which is for the protein of interest 110 and is labeled with the first single-stranded nucleic acid fragment 131, and the second specific binding substance 140 which is for the target protein 120 and is labeled with the second single-stranded nucleic acid fragment 141, into contact with the wells 241, and forming as a result the complex 100 comprising the protein of interest 110, the target protein 120, the first specific binding substance 130, and the second specific binding substance 140 when the protein of interest 110 binds to the target protein 120, with the double-stranded nucleic acid 150 being formed by hybridization of at least part of the first single-stranded nucleic acid fragment 131 with at least part of the second single-stranded nucleic acid fragment 141; and a step (b) of detecting formation of the double-stranded nucleic acid 150, wherein detection of formation of the double-stranded nucleic acid 150 indicates binding of the protein of interest 110 to the target protein 120.

In the case of the protein of interest 110 binding to the target protein 120, the above evaluation method may be the following detection method. Specifically, a detection method of the present embodiment may include bringing the protein of interest 110, the target protein 120, the first specific binding substance 130 which is for the protein of interest 110 and is labeled with the first single-stranded nucleic acid fragment 131, and the second specific binding substance 140 which is for the target protein 120 and is labeled with the second single-stranded nucleic acid fragment 141, into contact with the wells 241, binding the protein of interest 110 to the target protein 120, forming the complex 100 comprising the protein of interest 110, the target protein 120, the first specific binding substance 130, and the second specific binding substance 140, with the double-stranded nucleic acid 150 being formed by hybridization of at least part of the first single-stranded nucleic acid fragment 131 with at least part of the second single-stranded nucleic acid fragment 141, and detecting formation of the double-stranded nucleic acid 150.

According to the method of the present embodiment, mutant protein activity can be evaluated easily and in a short period of time. Mutant proteins may be proteins having VUS.

### <<Introduction process>>

First, as shown in Fig. 3, a reagent solution L210 is introduced from the inlet port 222 of the fluidic device 200 and supplied into the channel 230. The reagent solution L210 is a dispersion of a protein of interest 110, target protein 120, first specific binding substance 130, and second specific binding substance 140, and also contains a reagent for detecting formation of a double-stranded nucleic acid 150.

The reagent solution L210 supplied into the channel 230 comes into contact with the well array 240. The reagent solution L210 is accommodated inside the wells 241. Consequently, the protein of interest 110, target protein 120, first specific binding substance 130, second specific binding substance 140, and reagent for detecting formation of a double-stranded nucleic acid 150 are introduced into the wells 241. If the protein of interest 110 binds to the target protein 120, the reagent solution L210 contains a complex 100 comprising the protein of interest 110, target protein 120, first specific binding substance 130, and second specific binding substance 140.

The number of units of the complex 100 introduced into each well 241 is not specifically limited, but 1 or less, i.e., 0 or 1, unit of the complex 100 is preferred to be introduced into each well 241. Thus, units of the complex 100 can be detected on an individual basis, i.e., digital measurement can be performed. The complex 100 does not required to be introduced into all the wells of the well array.

The means of introducing the complex 100 into the wells is not specifically limited but may be a method, for example, in which the complex 100 is settled in the fluidic device (specifically, in the channel 230) by its own weight and distributed into the wells 241. Alternatively, a substance capturing the complex 100 (capture substance) may be used. Specifically, the complex 100 less likely to settle due to its own weight may be supplied with a capture substance bound thereto, or a capture substance may be immobilized in the wells in advance to capture the complex 100 supplied, so that the efficiency of introducing the complex 100 into the wells can be improved.

A capture substance can be bound to the complex 100 at any time during the method of the present embodiment. For example, before being introduced into the wells 241, the complex 100 may be brought into contact with the capture substance in a sample tube for binding. Alternatively, after introducing a capture substance into the wells 241, the complex 100 may be introduced into the wells for contact with the capture substance therein.

The capture substance is a substance capable of capturing the complex 100. The capture substance may be, for example, a conjugate of a solid phase and a specific binding substance for the complex 100.

The solid phase may be particles, membranes, substrates, or the like. One type, or two or more types of the specific binding substance may be used for the complex 100. For example, the number of the types of the specific binding substance may be three, four, or five or more.

The particles are not specifically limited, but may be polymer particles, magnetic particles, glass particles, or the like. The particles are preferred to undergo surface treatment to avoid nonspecific adsorption. In order to immobilize the specific binding substance, it is preferred to use particles having a functional group such as a carboxyl group on the surfaces. More specifically, Magnosphere LC300 (product name) manufactured by JSR Corporation, or the like can be used.

Examples of the first specific binding substance 130, the second specific binding substance 140, and the specific binding substance in the capture substance include antibodies, antibody fragments, and aptamers. Examples of the antibody fragments include Fab, F(ab')₂, Fab', single chain antibodies (scFvs), disulfide stabilized antibodies (dsFvs), dimeric V region fragments (diabodies), and peptides including CDRs. The antibodies may be monoclonal or polyclonal. Alternatively, the antibodies may be commercially available antibodies.

The method of labelling a specific binding substance with a single-stranded nucleic acid fragment may include methods using cross-linking agents. The specific binding substance may be labelled with a single-stranded nucleic acid fragment via a linker molecule. Examples of the linker include polyethylene chains, hydrocarbon chains, and peptides, but are not specifically limited thereto. The single-stranded nucleic acid fragment may be DNA or RNA. The single-stranded nucleic acid fragment may contain an artificial nucleic acid such as BNA or LNA.

The method of immobilizing the specific binding substance on particle surfaces is not specifically limited, but may be a method using physical adsorption, a method using chemical bonding, a method using avidin-biotin binding, a method using binding of protein G or protein A to an antibody, or the like. The method using physical adsorption may be a method of immobilizing the specific binding substance on particle surfaces using hydrophobic interaction or electrostatic interaction. The method using chemical bonding may be a method using a cross-linking agent. For example, if the particle surfaces have a hydroxyl group, the carboxyl group of the specific binding substance may be reacted with a cross-linking agent to obtain an active ester, and then the hydroxyl group and the ester group may be reacted with each other, so that the specific binding substance can be immobilized on the particle surfaces. It is preferred to provide spacers between the specific binding substance and the particle surfaces so as not to inhibit the ability of the specific binding substance to recognize the target molecules.

As described above, when introducing the complex 100 into the wells 241 using a capture substance, a conjugate of the capture substance and the complex 100 is preferred to be formed under the condition that 0 or 1 unit of the complex 100 is captured by 1 molecule of the capture substance. The wells 241 are preferred to be configured so that 0 or 1 molecule of the capture substance is introduced into each well 241. Thus, digital measurement can be performed.

If the protein of interest 110 binds to the target protein 120, when the protein of interest 110, target substance 120, first specific binding substance 130, and second specific binding substance 140 are brought into contact with each other, a complex 100 comprising these substances is formed, with a double-stranded nucleic acid 150 being formed by hybridization of at least part of the first single-stranded nucleic acid fragment 131 with at least part of the second single-stranded nucleic acid fragment 141. The complex 100 may be formed in a sample tube or in the wells 241.

### <<Sealing process>>

Following introduction of the reagent solution L210 into the wells 241, the openings of the wells 241 may be sealed. The method of sealing the openings of the wells 241 is not specifically limited as long as the liquid accommodated in each well 241 is not mixed with the liquid accommodated in another well 241. For example, the openings of the wells 241 may be sealed by covering them with a sealing liquid. Alternatively, the openings of the wells 241 may be sealed by laminating a plate-like member such as a glass plate on them.

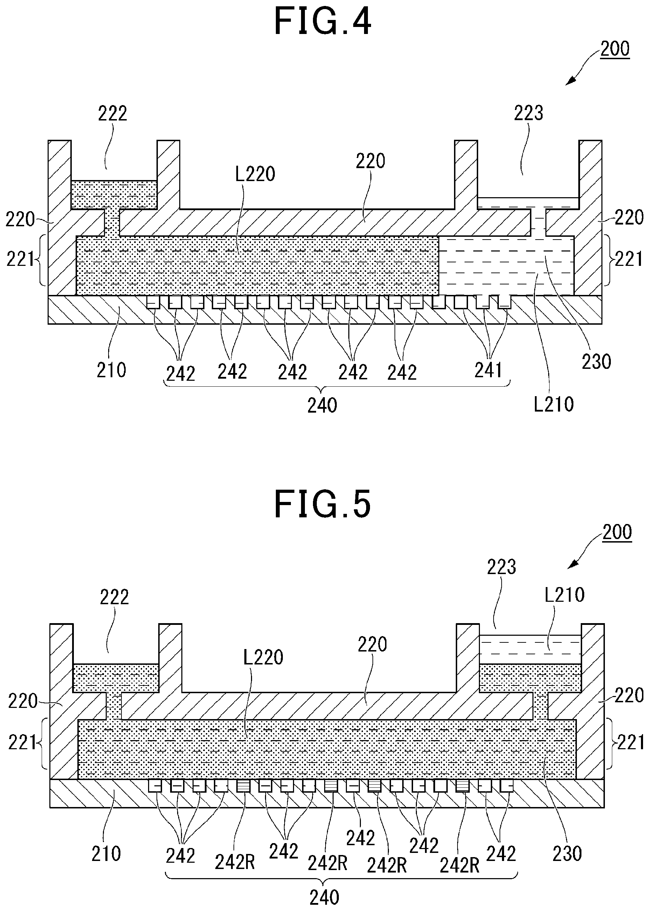

For example, as shown in Fig. 4, a sealing liquid L220 may be supplied into the channel 230 between the substrate 210 and the lid member 220 from the inlet port 222 of the lid member 220. The sealing liquid L220 supplied into the channel 230 may come into contact with the well array 240. The sealing liquid L220 may expel and replace the reagent solution L210 supplied into the channel 230 and is not accommodated in the wells 241. Thus, the sealing liquid L220 may individually seal the plurality of wells 241 in which the reagent solution L210 containing the target substance 110 is accommodated, and the wells 241 may become independent reaction spaces (also referred to as microcompartments 242). When the channel 230 is filled with the sealing liquid L220, excess sealing liquid L220 may be discharged from the outlet port 223. Fig. 5 shows a state in which all the wells 241 of the well array 240 are sealed with the sealing liquid L220, forming sealed wells (i.e., microcompartments) 242.

Alternatively, a lipid may be dissolved in the reagent solution L210 and, after supplying the sealing liquid L220 into the channel 230, the solution containing the lipid may be additionally supplied to form a lipid bilayer membrane at the openings of the wells 241, so that the plurality of wells 241 are individually sealed with the lipid bilayer membrane, forming sealed wells 242. Examples of the lipid to form the lipid bilayer membrane include 1,2-dioleoyl-sn-glycero-3-phosphoethanolamine (also referred to as DOPE), 1,2-dioleoyl-sn-glycero-3-phosphoglycerol (also referred to as DOPG), and mixtures thereof, but are not limited thereto.

The sealing liquid is a liquid that can form droplets (also referred to as microdroplets) by sealing the liquids introduced into the multiple wells 241 individually so that they do not mix with each other. The sealing solution is preferred to be an oily solution, and more preferred to be an oil. Examples of the oil that can be used include fluorine oils, silicone oils, hydrocarbon oils, and mixtures thereof. More specifically, FC-40 (product name) manufactured by Sigma-Aldrich, or the like can be used. FC-40 (CAS Number: 86508-42-1) is a fluorinated aliphatic compound with a specific gravity at 25°C being 1.85 g/mL.

### <<Detection process>>

Subsequently, formation of a double-stranded nucleic acid 150 is detected. The double-stranded nucleic acid 150 is preferred to be detected using a signal amplification reaction. For example, the signal amplification reaction may be an invasive cleavage assay (also referred to as ICA).

The ICA reaction relates to the principle that signal amplification progresses through two reaction cycles which are (1) complementary binding between nucleic acids and (2) recognition and cleavage of a triple-stranded structure by enzymes.

The ICA reaction is less affected by reaction cycle inhibition due to foreign substances. Accordingly, using the ICA reaction, formation of the double-stranded nucleic acid 150 can be detected with high accuracy. If the ICA reaction is used as the signal amplification reaction, the reagent solution L210 (i.e., solution containing a protein of interest 110, target protein 120, first specific binding substance 130, and second specific binding substance 140) may preferably contain a reaction reagent required for the ICA reaction.

Examples of the reaction reagent required for the ICA reaction include ICA reaction reagents such as flap probes, flap endonucleases (also referred to as FENs), and fluorescent substrates. Flap probes are nucleic acid fragments designed to hybridize with the first single-stranded nucleic acid fragment 131 or the second single-stranded nucleic acid fragment 141 to form a flap structure with the double-stranded nucleic acid 150.

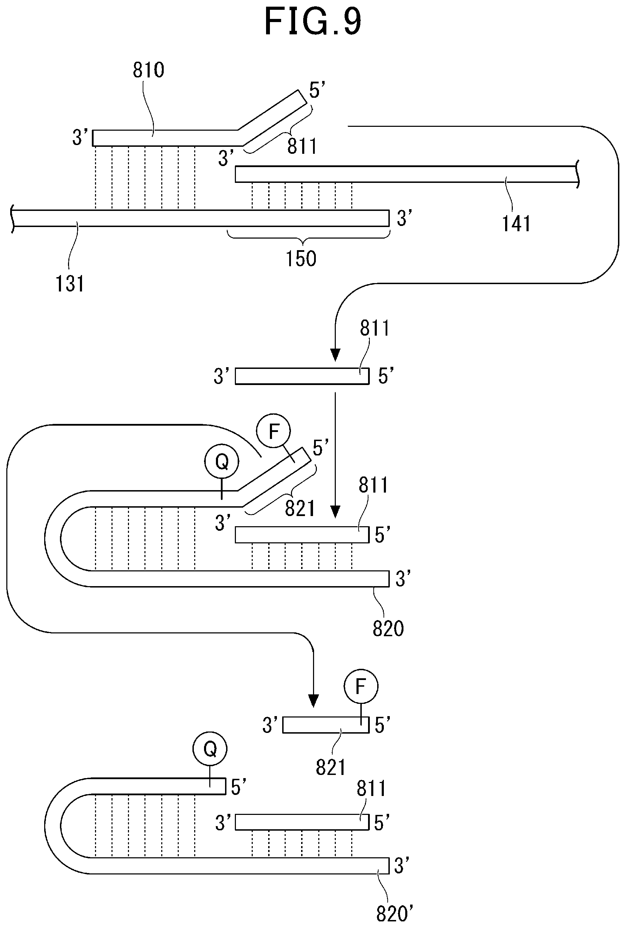

Fig. 9 is a schematic diagram illustrating an example of an ICA method. Fig. 9 shows an example of detecting a double-stranded nucleic acid 150 formed by hybridization of at least part of the first single-stranded nucleic acid fragment 131 with at least part of the second single-stranded nucleic acid fragment 141 using an ICA method.

First, a flap probe is hybridized with the first single-stranded nucleic acid fragment 131 or the second single-stranded nucleic acid fragment 141. In the example shown in Fig. 9, a flap probe 810 is hybridized with the first single-stranded nucleic acid fragment 131. Consequently, a first flap site 811 is formed.

Subsequently, an FEN is reacted with the first flap site 811, by which the first flap site 811 is cleaved to produce a nucleic acid fragment 811. Subsequently, the nucleic acid fragment 811 is hybridized with a fluorescent substrate (i.e., nucleic acid fragment 820) to form a second flap site 821.

In the example shown in Fig. 9, a fluorescent substance F is bound to the 5' end of the nucleic acid fragment 820, and a quenching substance Q is bound to the 3' side of the nucleic acid fragment 820 a few bases from the 5' end. Subsequently, the second flap site 821 is reacted with an FEN, by which the second flap site 821 is cleaved to produce a nucleic acid fragment 821. Consequently, the fluorescent substance F is separated from the quenching substance Q and generates a fluorescent signal. By detecting the fluorescent signal, formation of a double-stranded nucleic acid 150 can be detected.

The reagent solution L210 can be a liquid that is generally used in biochemical analyses performed using fluidic devices, and is preferred to be an aqueous solution. A surfactant or the like may be added to the reagent solution L210 to facilitate sealing of liquids in the wells.

When using the ICA reaction to detect formation of a double-stranded nucleic acid 150, if a double-stranded nucleic acid 150 is present, the fluorescent substance F is released from the quenching substance Q due to an isothermal enzymatic reaction, and a predetermined fluorescent signal is emitted in response to the excitation light.

For detecting formation of a double-stranded nucleic acid 150, a known suitable method can be selected according to the type of the signals to be detected. For example, when observing fluorescent signals, excitation light for the fluorescent substance may be applied to the sealed wells 242 to observe fluorescence emitted by the fluorescent substance. For example, as shown in Fig. 5, a predetermined reaction may be allowed to occur in the sealed wells 242, and generated signals may be observed. In Fig. 5, sealed wells 242R are wells where signals have been detected, and sealed wells 242 are wells where no signals have been detected.

Referring to Figs. 6 to 8, a method of the present embodiment will be described, taking an example of using the fluidic device 500.

First, as shown in Fig. 6, a reagent solution L210 is introduced into the fluidic device 500. The reagent solution L210 is a dispersion of a protein of interest 110, target protein 120, first specific binding substance 130, and second specific binding substance 140, and also contains a reagent for detecting formation of a double-stranded nucleic acid 150. If the protein of interest 110 binds to the target protein 120, the reagent solution L210 may contain a complex 100 comprising the protein of interest 110, target protein 120, first specific binding substance 130, and second specific binding substance 140. In the reagent solution L210, the concentration of the complex 100 is preferred to be adjusted such that 1 unit or less of the complex 100 is accommodated in each well 241.

Subsequently, as shown in Fig. 7, a sealing liquid L220 is introduced into the fluidic device 500. The specific gravity of the sealing liquid L220 is greater than that of the reagent solution L210. Therefore, the sealing liquid L220 sinks below the reagent solution L210 not accommodated in the wells 241 and contacts the well array 240. Thus, the sealing liquid L220 seals the plurality of wells 241 individually in which the reagent solution L210 containing the complex 100 is accommodated, and the wells 241 become independent reaction spaces (also referred to as microcompartments) 242.

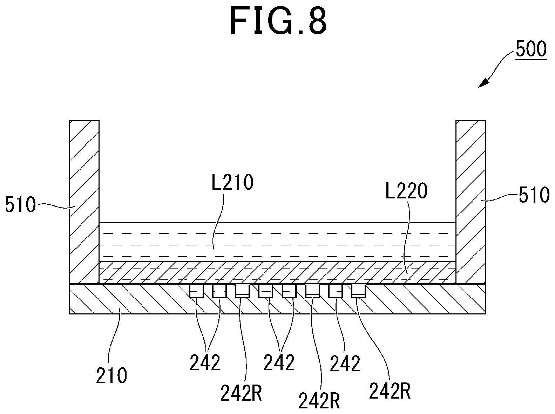

Subsequently, as shown in Fig. 8, a predetermined reaction is allowed to occur in the microcompartments 242, and generated signals are observed. In Fig. 8, microcompartments 242R are wells where signals have been detected, and microcompartments 242 are wells where no signals have been detected.

In the method of the present embodiment, step (a), that is, a step of bringing the protein of interest 110, the target protein 120, the first specific binding substance 130, and the second specific binding substance 140, into contact with each other in a container, and forming as a result the complex 100 comprising the protein of interest 110, the target protein 120, the first specific binding substance 130, and the second specific binding substance 140 when the protein of interest 110 binds to the target protein 120, with the double-stranded nucleic acid 150 being formed by hybridization of at least part of the first single-stranded nucleic acid fragment 131 with at least part of the second single-stranded nucleic acid fragment 141, may include a step (a1) of synthesizing the protein of interest 110 in the container using a cell-free protein synthesis system, and a step (a2) of bringing the protein of interest 110, the target protein 120, the first specific binding substance 130, and the second specific binding substance 140, into contact with each other in the container, and forming as a result the complex 100 comprising the protein of interest 110, the target protein 120, the first specific binding substance 130, and the second specific binding substance 140 when the protein of interest 110 binds to the target protein 120, with the double-stranded nucleic acid 150 being formed by hybridization of at least part of the first single-stranded nucleic acid fragment 131 with at least part of the second single-stranded nucleic acid fragment 141.

Specifically, the protein of interest 110 may be synthesized using a cell-free protein synthesis system, so that formation of the complex 100 can be performed sequentially with synthesis of the protein of interest 110. Thus, if the protein of interest is a kinase or the like containing a gene mutation, the protein of interest activity can be evaluated easily and in a short period of time.

The cell-free protein synthesis system refers to a synthesis system that synthesizes proteins in vitro from template nucleic acids using ribosomes, or transcription and translation factors, etc. derived from living cells or synthesized artificially, instead of synthesizing proteins within cells.

The cell-free protein synthesis system may be a synthesis system that includes a transcription process in addition to the translation process. If the nucleic acid encoding a protein is a DNA, it is necessary to synthesize an RNA encoding the protein by transcribing the DNA. In this case, the cell-free protein synthesis system may contain factors enabling transcription. Examples of the factors enabling transcription include RNA polymerase and nucleotides, but are not limited thereto, and can include factors known to those skilled in the art.

Alternatively, an RNA may be synthesized in advance using a DNA encoding a protein as a template, and the RNA may be added to a cell-free protein synthesis system. Alternatively, an artificially chemically synthesized RNA may be used.

The nucleic acid fragment serving as a template for cell-free protein synthesis may be bio-derived, cultured cell-derived, or virus-derived. Alternatively, the nucleic acid fragment may be artificially synthesized based on genetic analysis results.

The cell-free protein synthesis system is not specifically limited, but examples of which include synthesis systems using cell extracts obtained from wheat germ, yeast, insect cells, cultured mammalian cells, rabbit reticulocytes, or Escherichia coli, etc.; and synthesis systems reconstituting factors necessary for translation. Of these synthesis systems, cell-free protein synthesis in human expression system is preferred.

As factors related to translation, the cell-free protein synthesis system may contain, for example, at least one of tRNA, aminoacylated tRNA synthetase, translation initiation factors, translation elongation factors, and translation termination factors.

At step (a2) described above, adenosine triphosphate (ATP) may be additionally brought into contact with the protein of interest, target protein, first specific binding substance, and second specific binding substance. Thus, whether the target protein is phosphorylated can be evaluated. In other words, a kinase assay can be carried out.

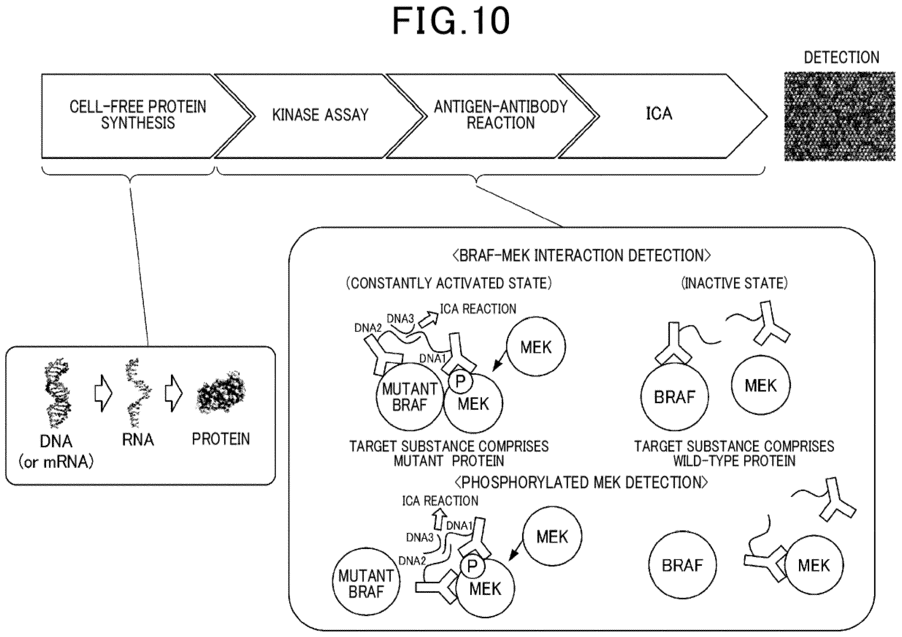

Fig. 10 is a schematic diagram illustrating an example of a method of the present embodiment. Herein, it is determined, as an example, that, when the mutant protein is BRAF, whether the variants of unknown significance in the BRAF gene brings about a constantly activated state.

In Fig. 10, a cell-free protein synthesis, kinase assay, antigen-antibody reaction, and ICA reaction are sequentially carried out. Thus, the protein of interest activity can be evaluated easily and in a short period of time. Also, for example, by adding an inhibitor to the reaction system, the effect of the inhibitor can also be evaluated.

In Fig. 10, the timing of adding a reagent required for each reaction and the timing of introducing the reagent solution into the fluidic device can be appropriately selected. For example, after performing the cell-free protein synthesis, reagents required for a kinase assay and antigen-antibody reaction may be added to perform these processes. An ICA reaction reagent may be added to the mixture resulting from the antigen-antibody reaction, and the resultant mixture may be introduced into the fluidic device to perform an ICA reaction. Alternatively, after performing a cell-free protein synthesis, reagents required for the kinase assay, antigen-antibody reaction, and ICA reaction may be added, and the resultant mixture may be introduced into the fluidic device to perform these processes. Alternatively, after performing a cell-free protein synthesis, reagents required for the kinase assay, antigen-antibody reaction, and ICA reaction may be added, followed by performing a kinase assay and antigen-antibody reaction, and then the resultant mixture may be introduced into the fluidic device to perform an ICA reaction.

As another method, after performing a cell-free protein synthesis, reagents required for the kinase assay and antigen-antibody reaction may be added to perform these processes. After that, a reagent required for the ICA reaction may be added, and the resultant mixture may be introduced into the fluidic device to perform an ICA reaction. Alternatively, after performing a cell-free protein synthesis, a reagent required for the kinase assay may be added to perform a kinase assay. After that, reagents required for the antigen-antibody reaction and ICA reaction may be added, followed by performing an antigen-antibody reaction, and the resultant mixture may be introduced into the fluidic device to perform an ICA reaction. To achieve good detection sensitivity, it is preferred that a reagent required for the ICA reaction is added following the kinase assay, and the resultant mixture is introduced into the fluidic device.

It is preferred that the method of the present embodiment does not include a cleaning process. In particular, in the case of synthesizing the protein of interest 110 using a cell-free protein synthesis system also, if the protein of interest can be evaluated as to whether it binds to the target protein without performing a cleaning process, the protein of interest can be evaluated even more easily and in a shorter period of time.

### [Complex]

In an embodiment, the present invention provides the complex 100 comprising the protein of interest 110, the target protein 120, the first specific binding substance 130 which is for the protein of interest 110 and is labeled with the first single-stranded nucleic acid fragment 131, and the second specific binding substance 140 which is for the target protein 120 and is labeled with the second single-stranded nucleic acid fragment 141, with the double-stranded nucleic acid 150 being formed by hybridization of at least part of the first single-stranded nucleic acid fragment 131 with at least part of the second single-stranded nucleic acid fragment 141.

In the complex 100, a flap probe may be further hybridized with the first single-stranded nucleic acid fragment 131 or the second single-stranded nucleic acid fragment 141.

As described above, using the complex of the present embodiment, mutant protein activity can be evaluated easily and in a short period of time.

### [Kit]

In an embodiment, the present invention provides a kit for evaluating whether the protein of interest 110 binds to the target protein 120, the kit including the well array 240 having the plurality of wells 241, the first specific binding substance 130 which is for the protein of interest 110 and is labeled with the first single-stranded nucleic acid fragment 131, and the second specific binding substance 140 which is for the target protein 120 and is labeled with the second single-stranded nucleic acid fragment 141.

Using the kit of the present embodiment, whether the protein of interest 110 binds to the target protein 120 can be preferably evaluated.

Accordingly, the kit of the present embodiment can be said to be used for the method including a step of introducing the protein of interest 110, the target protein 120, the first specific binding substance 130 which is for the protein of interest 110 and is labeled with the first single-stranded nucleic acid fragment 131, and the second specific binding substance 140 which is for the target protein 120 and is labeled with the second single-stranded nucleic acid fragment 141, into the wells 241, and forming as a result the complex 100 comprising the protein of interest 110, the target protein 120, the first specific binding substance 130, and the second specific binding substance 140 when the protein of interest 110 binds to the target protein 120, with the double-stranded nucleic acid 150 being formed by hybridization of at least part of the first single-stranded nucleic acid fragment 131 with at least part of the second single-stranded nucleic acid fragment 141, and a step of detecting formation of the double-stranded nucleic acid 150, wherein detection of formation of the double-stranded nucleic acid 150 indicates binding of the protein of interest 110 to the target protein 120.

The well array may be disposed inside the fluidic device described above. In the kit of the present embodiment, the protein of interest, the target protein, the first single-stranded nucleic acid fragment, the first specific binding substance, the second single-stranded nucleic acid fragment, and the second specific binding substance are similar to those which are described above.

The kit of the present embodiment may further include ATP. Thus, whether the target protein is phosphorylated can be evaluated. In other words, a kinase assay can be carried out.

The kit of the present embodiment may further include a sealing liquid L220 for sealing the openings of the wells 241. The sealing liquid L220 is similar to the sealing liquid described above.

The kit of the present embodiment may further include a reagent for detecting the double-stranded nucleic acid 150 formed by hybridization of at least part of the first single-stranded nucleic acid fragment 131 with at least part of the second single-stranded nucleic acid fragment 141.

Examples of such a reagent include the ICA reaction reagents mentioned above, specifically including flap probes, flap endonucleases, and fluorescent substrates.

### [Method of evaluating whether protein of interest phosphorylates target protein]

**In** an embodiment, the present invention provides a method of evaluating whether a protein of interest phosphorylates a target protein, the method including a step of bringing the protein of interest, the target protein, the second specific binding substance which is for the target protein and is labeled with the second single-stranded nucleic acid fragment, a third specific binding substance which is for the target protein undergone phosphorylation and is labeled with a third single-stranded nucleic acid fragment, and ATP, into contact with each other in a container, and forming as a result a complex comprising the target protein, the second specific binding substance, and the third specific binding substance when the target protein is phosphorylated, with a double-stranded nucleic acid being formed by hybridization of at least part of the second single-stranded nucleic acid fragment with at least part of the third single-stranded nucleic acid fragment, and a step of detecting formation of the double-stranded nucleic acid, wherein detection of formation of the double-stranded nucleic acid indicates phosphorylation of the target protein.

When the protein of interest phosphorylates the target protein, the above evaluation method may be the following detection method. Specifically, the detection method of the present embodiment may include bringing the protein of interest, the target protein, the second specific binding substance which is for the target protein and is labeled with the second single-stranded nucleic acid fragment, the third specific binding substance which is for the target protein undergone phosphorylation and is labeled with the third single-stranded nucleic acid fragment, and ATP, into contact with each other in a container, forming the complex comprising the target protein, the second specific binding substance, and the third specific binding substance, with the double-stranded nucleic acid being formed by hybridization of at least part of the second single-stranded nucleic acid fragment with at least part of the third single-stranded nucleic acid fragment, and detecting formation of the double-stranded nucleic acid.

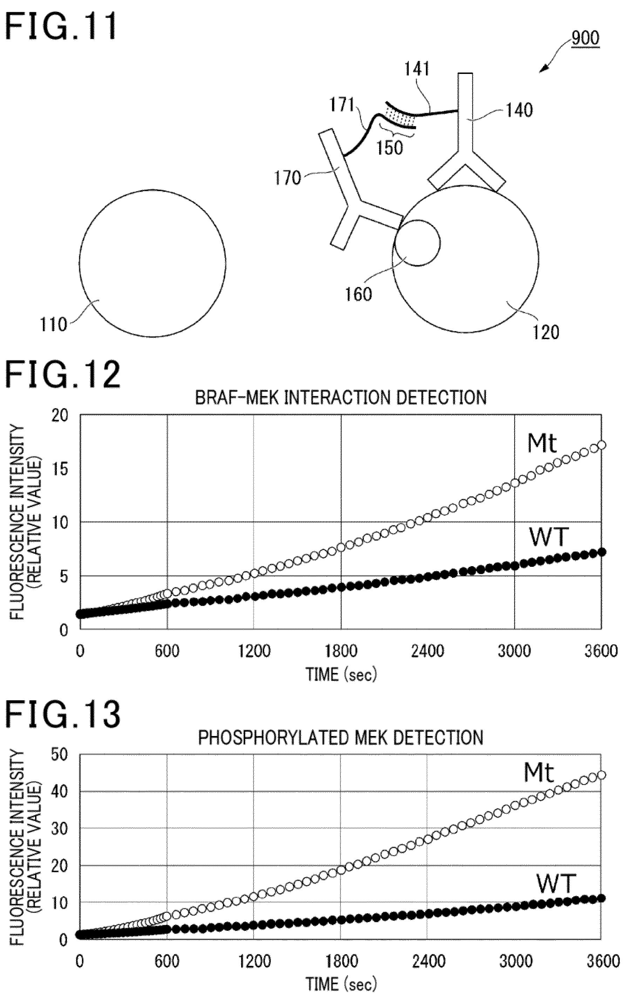

Fig. 11 is a schematic diagram illustrating the method of the present embodiment. As shown in Fig. 11, in the method of the present embodiment, at step (a') first, the protein of interest 110, the target protein 120, the second specific binding substance 140 which is for the target protein 120 and is labeled with the second single-stranded nucleic acid fragment 141, and a third specific binding substance 170 which is for the target protein 120 undergone phosphorylation (the phosphate group is indicated by reference sign 160 in Fig. 11) and is labeled with a third single-stranded nucleic acid fragment 171, and ATP (not shown) are brought into contact with each other in a container.

As a consequence, if the target protein 120 is phosphorylated due to the kinase activity of the protein of interest 110, a complex 900 is formed comprising the target protein 120, the second specific binding substance 140, and the third specific binding substance 170, with a double-stranded nucleic acid (also referred to as double-stranded nucleic acid region) 150 being formed by hybridization of at least part of the second single-stranded nucleic acid fragment 141 with at least part of the third single-stranded nucleic acid fragment 171.

Step (a') is similar to step (a) in the method of evaluating whether the protein of interest binds to the target protein described above; however, is mainly different in that the third specific binding substance 170 is brought into contact instead of the first specific binding substance 130.

Subsequently, in step (b), formation of the double-stranded nucleic acid 150 is detected. If formation of the double-stranded nucleic acid 150 is detected, it can be determined that the target protein 120 has been phosphorylated. Step (b) is similar to step (b) in the method of evaluating whether the protein of interest binds to the target protein described above. In the method of the present embodiment, the amount of the double-stranded nucleic acid 150 formed corresponds to the amount of the target protein 120 present after being phosphorylated.

The method of the present embodiment may be applied to the case where the protein of interest 110 does not phosphorylate the target protein 120. In that case, formation of the double-stranded nucleic acid 150 is not detected.

As will be described later in examples, according to the present invention, mutant protein activity can be evaluated easily and in a short period of time. For example, a functional analysis (i.e., evaluation) of VUS can be performed easily, efficiently, and in a short period of time.

If the protein of interest 110 exerts kinase activity on the target protein 120, it is considered that the protein of interest 110 binds to the target protein 120, first, to form the complex 900, and then the target protein 120 is phosphorylated due to the kinase activity of the protein of interest 110.

Accordingly, the functions of the protein of interest 110 can be analyzed (i.e., evaluated) using the method of evaluating whether the protein of interest binds to the target protein described above, or the method of the present embodiment.

From the perspective that at least part of the second single-stranded nucleic acid fragment 141 is required to be able to hybridize with at least part of the third single-stranded nucleic acid fragment 171, the second single-stranded nucleic acid fragment 141 may have a length of 10 to 200 bases. Similarly, the third single-stranded nucleic acid fragment 171 may also have a length of 10 to 200 bases. The double-stranded nucleic acid 150 formed by hybridization of at least part of the second single-stranded nucleic acid fragment 141 with at least part of the third single-stranded nucleic acid fragment 171 is preferred to have a length of approximately 7 to 30 bases, e.g., may be 9, 12 or 15 bases.

In the method of the present embodiment, the protein of interest 110 may be a kinase having variants of unknown significance. More specific examples of the protein of interest may be proteins in intracellular signal transduction pathways, including, for example, BRAF, A-RAF, Raf, MAP3K 4/12, MAP3K11, ASK1, and TAK1. In this case, whether the protein having VUS is in a constantly activated state can be evaluated based on whether the protein of interest phosphorylates the target protein.

In an embodiment, the present invention provides a kit for evaluating whether the protein of interest 110 phosphorylates the target protein 120, the kit including the well array 240 having the plurality of wells 241, the second specific binding substance 140 which is for the target protein 120 and is labeled with the second single-stranded nucleic acid fragment 141, the third specific binding substance 170 which is for the target protein 120 undergone phosphorylation and is labeled with the third single-stranded nucleic acid fragment 171, and ATP.

Using the kit of the present embodiment, whether the protein of interest 110 phosphorylates the target protein 120 can be preferably evaluated.

Accordingly, the kit of the present embodiment can be said to be used for the method including a step of introducing the protein of interest 110, the target protein 120, the second specific binding substance 140 which is for the target protein 120 and is labeled with the second single-stranded nucleic acid fragment 141, the third specific binding substance 170 which is for the target protein 120 undergone phosphorylation and is labeled with the third single-stranded nucleic acid fragment 171, and ATP, into the wells 241, and forming as a result the complex 900 comprising the target protein 120, the second specific binding substance 140, and the third specific binding substance 170 when the protein of interest 110 phosphorylates the target protein 120, with the double-stranded nucleic acid 150 being formed by hybridization of at least part of the second single-stranded nucleic acid fragment 141 with at least part of the third single-stranded nucleic acid fragment 171, and a step of detecting formation of the double-stranded nucleic acid 150, wherein detection of formation of the double-stranded nucleic acid 150 indicates the protein of interest 110 phosphorylating the target protein 120.

The well array may be disposed inside the fluidic device described above. In the kit of the present embodiment, the protein of interest, the target protein, the second single-stranded nucleic acid fragment, the second specific binding substance, the third single-stranded nucleic acid fragment, and the third specific binding substance are similar to those which are described above.

The kit of the present embodiment may further include a sealing liquid L220 for sealing the openings of the wells 241. The sealing liquid L220 is similar to the sealing liquid described above.

The kit of the present embodiment may further include a reagent for detecting the double-stranded nucleic acid 150 formed by hybridization of at least part of the second single-stranded nucleic acid fragment 141 with at least part of the third single-stranded nucleic acid fragment 171.

Examples of such a reagent include the ICA reaction reagents mentioned above, specifically including flap probes, flap endonucleases, and fluorescent substrates.

The present invention includes other aspects as follows.
[1] A detection method including
   bringing a protein of interest, a target protein, a first specific binding substance which is for the protein of interest and is labeled with a first single-stranded nucleic acid fragment, and a second specific binding substance which is for the target protein and is labeled with a second single-stranded nucleic acid fragment, into contact with each other in a container;
   binding the protein of interest to the target protein to form a complex comprising the protein of interest, the target protein, the first specific binding substance, and the second specific binding substance;
   forming a double-stranded nucleic acid by hybridization of at least part of the first single-stranded nucleic acid fragment with at least part of the second single-stranded nucleic acid fragment; and
   detecting formation of the double-stranded nucleic acid.
[2] The detection method according to [1], further including synthesizing the protein of interest using a cell-free protein synthesis system in the container.
[3] The detection method according to [2], wherein forming the double-stranded nucleic acid includes additionally bringing adenosine triphosphate into contact with the protein of interest, the target protein, the first specific binding substance, and the second specific binding substance.
[4] The detection method according to any one of [1] to [3], wherein the method does not include any process.
[5] The detection method according to any of [1] to [4], wherein the container comprises wells, and each of the wells have a volume of 10 fL to 100 pL.
[6] The detection method according to any of [1] to [5], wherein the first single-stranded nucleic acid fragment and the second single-stranded nucleic acid fragment each have a length of 10 to 200 bases.
[7] The detection method according to any of [1] to [6], further including adding a reagent for detecting formation of the double-stranded nucleic acid to the container after formation of the complex but before formation of the double-stranded nucleic acid.
[8] The detection method according to any of [1] to [7], wherein detecting formation of the double-stranded nucleic acid is performed using an invasive cleavage assay.
[9] A detection method including
   bringing a protein of interest, a target protein, a second specific binding substance which is for the target protein and is labeled with a second single-stranded nucleic acid fragment, a third specific binding substance which is for the target protein undergone phosphorylation and is labeled with a third single-stranded nucleic acid fragment, and ATP, into contact with each other in a container;
   phosphorylating the target protein to form a complex comprising the target protein, the second specific binding substance, and the third specific binding substance;
   forming a double-stranded nucleic acid by hybridization of at least part of the second single-stranded nucleic acid fragment with at least part of the third single-stranded nucleic acid fragment; and
   detecting formation of the double-stranded nucleic acid.
[10] The detection method according to [9], further including synthesizing the protein of interest using a cell-free protein synthesis system in the container.
[11] The detection method according to [9] or [10], wherein the method does not include any cleaning process.
[12] The detection method according to [9] or [10], wherein the container comprises wells, and each of the wells has a volume of 10 fL to 100 pL.
[13] The detection method according to any of [9] to [12], wherein the second single-stranded nucleic acid fragment and the third single-stranded nucleic acid fragment each have a length of 10 to 200 bases.
[14] The detection method according to any of [9] to [13], further including adding a reagent for detecting formation of the double-stranded nucleic acid to the container after formation of the complex but before formation of the double-stranded nucleic acid.
[15] The detection method according to any of [9] to [14], wherein detecting formation of the double-stranded nucleic acid is performed using an invasive cleavage assay.

### Examples

### [Materials and methods]

### (Oligonucleotide modification to antibody <<BRAF-MEK interaction detection>>)

Using an antibody-oligonucleotide conjugation tool (product name: oYo-Link antibody labeling reagent, manufactured by Funakoshi Co., Ltd.), different oligonucleotides (manufactured by Integrated DNA Technologies) were respectively bound to an anti-phosphorylation MEK rabbit polyclonal antibody (pMEK1/2 (S217/221) rabbit antibody, manufactured by Cell Signaling Technology, Inc.) and an anti-BRAF rabbit polyclonal antibody (manufactured by Proteintech). Specifically, oligonucleotides, i.e., DNA 1 (5'-TTTGTCACTGTTCCTCCTTTTGTTTTCCTTTCTGTGAGCAATTTCACCCAA-3', sequence number 1) and DNA 2 (5'-GCATGGTTCCAATTTGGGTGAT-3', sequence number 2), were respectively bound to the anti-phosphorylation MEK rabbit polyclonal antibody and the anti-BRAF rabbit polyclonal antibody mentioned above.

### (Oligonucleotide modification to antibodies <<Phosphorylated MEK detection>>)

Using an antibody-oligonucleotide conjugation tool (product name: oYo-Link antibody labeling reagent, manufactured by Funakoshi Co., Ltd.), different oligonucleotides (manufactured by Integrated DNA Technologies) were respectively bound to an anti-phosphorylation MEK rabbit polyclonal antibody (pMEK1/2 (S217/221) rabbit antibody, manufactured by Cell Signaling Technology, Inc.) and an anti-MEK rabbit monoclonal antibody (MEK1 Detector Antibody, manufactured by Abcam). Specifically, oligonucleotides, i.e., DNA 1 (5'-TTTGTCACTGTTCCTCCTTTTGTTTTCCTTTCTGTGAGCAATTTCACCCAA-3', sequence number 1) and DNA 2 (5'-GCATGGTTCCAATTTGGGTGAT-3', sequence number 2), were respectively bound to the anti-phosphorylation MEK rabbit polyclonal antibody and the anti-MEK rabbit polyclonal antibody mentioned above.

### (Synthesis of mutant BRAF and wild-type BRAF using cell-free protein synthesis system)

A plasmid DNA (vector: pT7CFE1-CHis Expression Vector, manufactured by Thermo Fisher Scientific) carrying wild-type BRAF gene or mutant BRAF gene with variants of unknown significance, as a template, was added to a human cell-free protein synthesis system (1-Step Human Coupled IVT Kit-DNA, (manufactured by Thermo Fisher Scientific), or Human Cell-Free Protein Expression System (manufactured by Takara Bio)), and protein synthesis was performed at 30 to 32°C for 90 minutes to 6 hours to obtain a BRAF protein.

### (Kinase assay and antigen-antibody reaction)

Mixtures were each prepared by mixing a protein of interest (mutant BRAF or wild-type BRAF), a target protein (inactive MEK), the above two types of oligonucleotide-modified antibodies, ATP, and a blocking buffer each other. The volume of each mixture used was 10 µL. The protein of interest was prepared to have final concentrations of 0 pM, 2.82 pM, 28.2 pM, and 2,821 pM. The target protein was prepared to have a final concentration of 28,210 pM. The two types of oligonucleotide-modified antibodies were each prepared to have a final concentration of 8.56 nM. As the blocking buffer, a tris buffered saline (also referred to as TBS) containing 1% bovine serum albumin (also referred to as BSA) was used. Subsequently, each mixture was allowed to undergo a reaction at 30°C for 45 minutes, and then at 37°C for 60 minutes.

### (Preparation of invasive cleavage assay reaction reagent)

An ICA reaction reagent for detection was prepared to perform an ICA reaction using the oligonucleotides modified to the above antibodies. The ICA reaction reagent in the present experimental example contained 2 µM allele probe (5'-CGCGCCGAGGAATTGCTCACAGAAAGGA-3') (manufactured by FASMAC, sequence number 3), 4 µM FRET cassette 1 (fluorescent substrate, Alexa 488-BHQ: 5'-X-TTCT-Y-AGCCGGTTTTCCGGCTGAGACCTCGGCGCG-3', X: Alexa 488 + Amino C6, Y: Black hole quencher (BHQ) 1-dT) (manufactured by Japan Bio Services, sequence number 4), 0.108 µM to 1.73 µM flap endonuclease (FEN)-1, 50 mM Tris-HCl (pH 8.5), 20 mM MgCl₂, and 0.05% Tween 20. The concentrations of the components in the ICA reaction reagent are the final concentrations in the experimental examples.

### [Experimental Example 1]

### (Kinase activity evaluation for proteins of interest (mutant BRAF and wild-type BRAF) in tube)

10 µL of each mixture after the antigen-antibody reaction was mixed with 10 µL of the above ICA reaction reagent to undergo a reaction at 66°C for 1 hour using Rotor-Gene Q (manufactured by QIAGEN) to detect Alexa 488 fluorescence. This reaction may hereinafter be referred to as an immuno-ICA reaction.

Figs. 12 to 14 are graphs showing the results of Experimental Example 1. Fig. 12 shows the results of detecting interactions (i.e., binding) between BRAF and phosphorylated MEK by allowing an immuno-ICA reaction to occur using the mutant BRAF (Mt) or the wild-type BRAF (WT) at a concentration of 2,821 pM as a protein of interest. In Fig. 12, the vertical axis indicates Alexa 488 fluorescence intensity (relative value), and the horizontal axis indicates time (second) after start of ICA reaction.

Consequently, it was revealed that the interactions between the mutant BRAF and phosphorylated MEK could be detected. With the fluorescence intensity at the point of detecting an interaction between the wild-type BRAF and phosphorylated MEK being N (noise), and with the fluorescence intensity at the point of detecting an interaction between the mutant BRAF and phosphorylated MIK being S (signal), a signal-to-noise ratio (S/N ratio) at the end point was calculated, which produced a result of 2.4.

Fig. 13 shows the results of detecting phosphorylated MEK by allowing an immuno-ICA reaction to occur using the mutant BRAF (Mt) or the wild-type BRAF (WT) at a concentration of 2,821 pM as a protein of interest. In Fig. 13, the vertical axis indicates Alexa 488 fluorescence intensity (relative value), and the horizontal axis indicates time (second) after start of ICA reaction.

Consequently, it was revealed that the kinase activity of the mutant BRAF could be detected. With the fluorescence intensity at the point of detecting phosphorylated MEK in the case of reacting the wild-type BRAF being N (noise), and with the fluorescence intensity at the point of detecting phosphorylated MEK in the case of reacting the mutant BRAF being S (signal), a signal-to-noise ratio (S/N ratio) at the end point was calculated, which produced a result of 3.9.

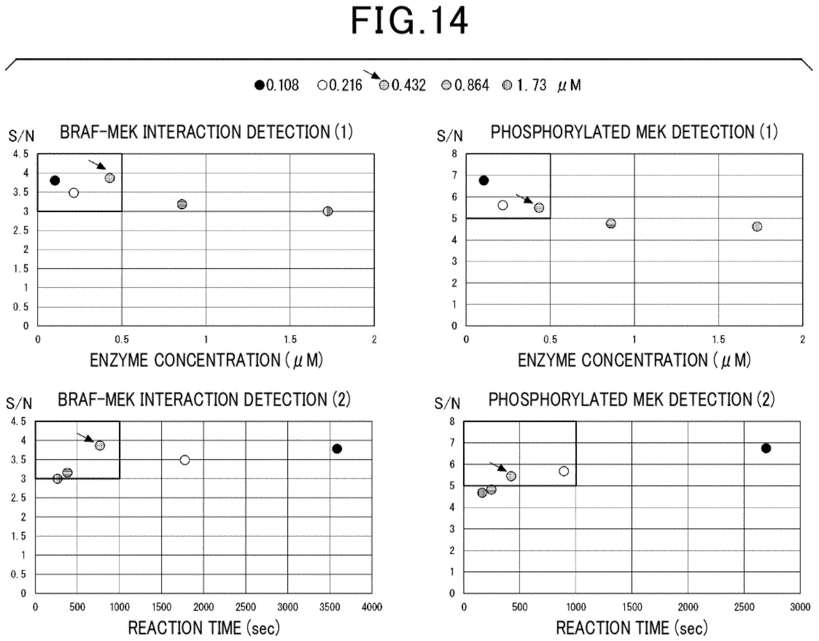

Fig. 14 is a set of graphs showing the results of calculating signal-to-noise ratios (S/N ratios) in the results shown in Figs. 12 and 13, with the fluorescence intensity at the antigen concentration of 0 nM being N (noise), and the fluorescence intensity at other concentrations being S (signal). In Fig. 14, the vertical axis indicates maximum S/N ratio in each reaction, and the horizontal axis indicates time (second) after start of ICA reaction or enzyme concentration. Consequently, the enzyme concentration of 0.432 µM was found to be the condition allowing a reaction to occur in a short reaction time and at a low enzyme concentration. The maximum S/N ratio in that case was 3.9 in the BRAF-MEK interaction detection, and 5.4 in the phosphorylated MEK detection.

### [Experimental Example 2]

### (Kinase activity evaluation for proteins of interest (mutant BRAF and wild-type BRAF) using fluidic device 1)

The interactions (i.e., binding) between the mutant BRAF and phosphorylated MEK and the interactions between the wild-type BRAF and phosphorylated MEK were detected as in Experimental Example 1, except that the fluidic devices as shown in Figs. 3 to 5 were used.

10 µL of each mixture after the antigen-antibody reaction was mixed with 10 µL of the above ICA reaction reagent, and the mixture was introduced into a fluidic device including a well array. The well array had approximately 1,000,000 wells, and each well had a volume of approximately 60 fL.

Subsequently, a sealing liquid (FC-40, CAS No.: 86508-42-1) was introduced into the fluidic device. As a consequence, the wells of the well array were individually sealed, providing independent reaction spaces.

Subsequently, the fluidic device was set in an aluminum block bath (model DTU-Mini manufactured by TAITEC Corporation), followed by heating at 66°C for 7 minutes or 30 minutes, and then observed using a microscope (product name: All-in-One fluorescence microscope, model BZ-X810 manufactured by KEYENCE Corporation). Subsequently, the luminance of each well was calculated based on the microscopic observation image.

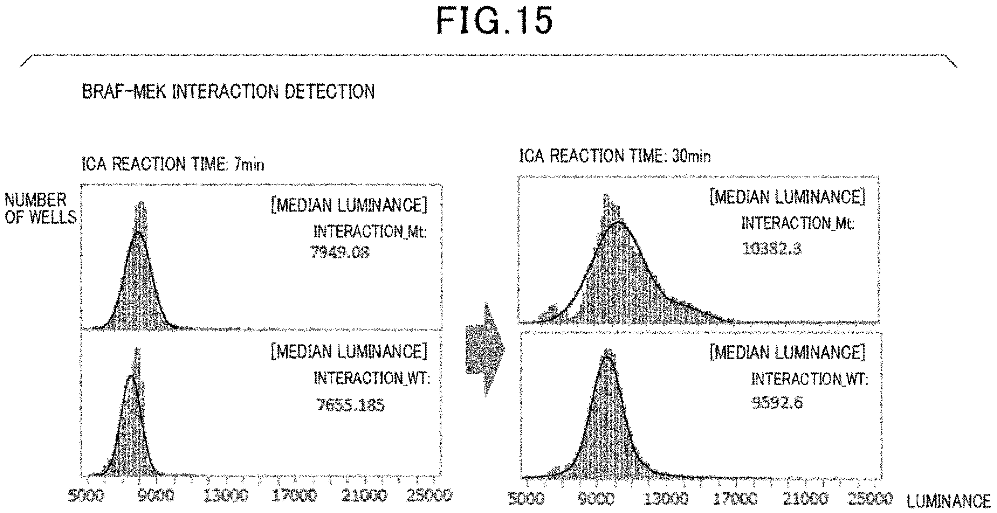

Fig. 15 is a set of graphs showing the calculation results for the luminance of the wells. Fig. 15 shows the results of detecting interactions between BRAF and phosphorylated MEK by allowing an immuno-ICA reaction to occur using the mutant BRAF (Mt) or the wild-type BRAF (WT), as a protein of interest. In Fig. 15, the vertical axis indicates the number of wells, and the horizontal axis indicates luminance. Fig. 15. shows, on the left, the results of 7-minute ICA reaction, and Fig. 15 shows, on the right, the results of 30-minute ICA reaction. Fig. 15 shows, on the upper part, the results of detecting the interactions between the mutant BRAF (Mt) and phosphorylated MEK, and Fig. 15 shows, on the lower part, the results of detecting the interactions between the wild-type BRAF (WT) and phosphorylated MEK.

Table 1 shown below summarizes the median luminance of the wells shown in Fig. 15. In table 1, S/N indicates a signal-to-noise ratio (S/N ratio) at the end point as calculated, where N (noise) represents the fluorescence intensity at the point of detecting an interaction between the wild-type BRAF and phosphorylated MEK, and S (signal) represents the fluorescence intensity at the point of detecting an interaction between the mutant BRAF and phosphorylated MEK.

**[Table 1]**

| | 7 min. | 30 min. | Difference |
|---|---|---|---|
| Mutant BRAF (Mt) | 7949.08 | 10382.3 | 2532.09 |
| Wild-type BRAF (WT) | 7655.185 | 9592.6 | 1968.865 |
| Difference | 293.895 | 857.12 | - |
| S/N | 1.038392 | 1.08906 | - |

As a consequence, it was confirmed that there were differences between the interactions between the mutant BRAF and phosphorylated MEK and the interactions between the wild-type BRAF and phosphorylated MEK, and it was clear that the interactions between the mutant BRAF and phosphorylated MEK could be detected.

### [Experimental Example 3]

### (Kinase activity evaluation for proteins of interest (mutant BRAF and wild-type BRAF) using fluidic device 2)

Phosphorylated MEK was detected as in Experimental Example 1, except that the fluidic devices as shown in Fig. 3 to 5 were used. The mutant BRAF and the wild-type BRAF were used as protein of interests.

10 µL of each mixture after the antigen-antibody reaction was mixed with 10 µL of the above ICA reaction reagent, and the mixture was introduced into a fluidic device including a well array. The well array had approximately 1,000,000 wells, and each well had a volume of approximately 60 fL.

Subsequently, a sealing liquid (FC-40, CAS No.: 86508-42-1) was introduced into the fluidic device. As a consequence, the wells of the well array were individually sealed, providing independent reaction spaces.

Subsequently, the fluidic device was set in an aluminum block bath (model DTU-Mini manufactured by TAITEC Corporation), followed by heating at 66°C for 7 minutes or 30 minutes, and then observed using a microscope (product name: All-in-One fluorescence microscope, model BZ-X810 manufactured by KEYENCE Corporation). Subsequently, the luminance of each well was calculated based on the microscopic observation image.

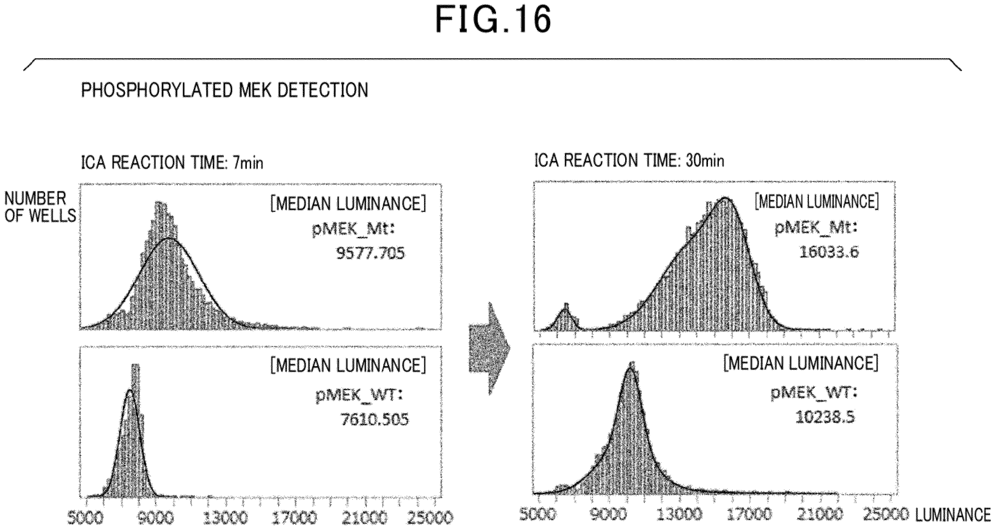

Fig. 16 is a set of graphs showing the calculation results for the luminance of the wells. Fig. 16 shows the results of detecting phosphorylated MEK by allowing an immuno-ICA reaction to occur using the mutant BRAF (Mt) or the wild-type BRAF (WT) as a protein of interest. In Fig. 16, the vertical axis indicates the number of wells, and the horizontal axis indicates luminance. Fig. 16. shows, on the left, the results of 7-minute ICA reaction, and Fig. 16 shows, on the right, the results of 30-minute ICA reaction. Fig. 16 shows, on the upper part, the results of detecting phosphorylated MEK in the case of reacting the mutant BRAF (Mt), and Fig. 16 shows, on the lower part, the results of detecting phosphorylated MEK in the case of reacting the wild-type BRAF (WT).

Table 2 shown below summarizes the median luminance of the wells shown in Fig. 16. In table 2, S/N indicates a signal-to-noise ratio (S/N ratio) at the end point as calculated, where N (noise) represents the fluorescence intensity in the case of reacting the wild-type BRAF, and S (signal) represents the fluorescence intensity in the case of reacting the mutant BRAF.

**[Table 2]**

| | 7 min. | 30 min. | Difference |
|---|---|---|---|
| Mutant BRAF (Mt) | 9577.705 | 16033.6 | 6455.895 |
| Wild-type BRAF (WT) | 7610.505 | 10238.5 | 2627.995 |
| Difference | 1967.2 | 5795.1 | - |
| S/N | 1.258485 | 1.566011 | - |

As a consequence, it was confirmed that there were differences between the amount of the phosphorylated MEK present in the case of reacting the mutant BRAF and the amount of the phosphorylated MEK present in the case of reacting the wild-type BRAF, and it was clear that kinase activity of BRAF could be detected.

### [Experimental Example 4]

### (Kinase activity evaluation for proteins of interest (mutant BRAF and wild-type BRAF) in tube 3)

Differences in activity depending on the order of adding reagents were evaluated. Pattern 1 is defined to the case of detecting phosphorylated MEK by mixing all the reagents for performing a kinase assay reaction, antigen-antibody reaction, and immuno-ICA reaction each other before performing a kinase assay. Pattern 2 is defined to be the case of detecting phosphorylated MEK by mixing the reagents for performing an antigen-antibody reaction and immuno-ICA reaction each other and introducing the mixture into a tube after performing a kinase assay.

The specific procedure of Pattern 1 will be described. Mixtures were each prepared by mixing a protein of interest (mutant BRAF or wild-type BRAF), a target protein (inactive MEK or active MEK), the above two types of oligonucleotide-modified antibodies, ATP, and a blocking buffer each other. The volume of each mixture used was 10 µL. The proteins of interest were each prepared to have a final concentration of 2,821 pM. The target proteins was each prepared to have a final concentration of 7 nM. The two types of oligonucleotide-modified antibodies were each prepared to have a final concentration of 1 nM when bound to DNA1, and to have a final concentration of 4 nM when bound to DNA2. As the blocking buffer, a tris buffered saline (also referred to as TBS) containing casein and a nonionic surfactant was used. As shown in Table 3, 6-pattern combinations of BRAF, MEK and ATP were used as reaction compositions. In Table 3, Good indicates that the relevant component is contained, and Poor indicates that the relevant component is not contained.

**[Table 3]**

| | BRAF | MEK | ATP |
|---|---|---|---|
| 1 | Wt | Active | Good |
| 2 | Poor (solution A) | Inactive | Good |
| 3 | Wt | Inactive | Good |
| 4 | Wt | Inactive | Poor |
| 5 | Mt | Inactive | Good |
| 6 | Mt | Inactive | Poor |

### (Preparation of ICA reaction reagents)

The ICA reaction reagent in Pattern 1 was prepared with the same composition and final concentration as those of Experimental Examples 1 to 3, except that 0.108 µM flap endonuclease (FEN)-1 was used. The concentrations of the components in the ICA reaction reagent are the final concentrations in the experimental examples.

A kinase assay, antigen-antibody reaction, and ICA reaction were performed in tube. Each mixture was mixed with the ICA reaction reagent and introduced into a tube. Following reaction at 30°C for 60 minutes, phosphorylated MEK was detected as in Experimental Example 1 (Pattern 1). The time of the immuno-ICA reaction was set to be within 60 minutes to 180 minutes.

The specific procedure of Pattern 2 will be described. Mixtures were each prepared by mixing a protein of interest (mutant BRAF or wild-type BRAF), a target protein (inactive MEK or active MEK), ATP, 20 mM MgCl₂, and a blocking buffer each other. The volume of each mixture used was 7.5 µL. The proteins of interest were each prepared to have a final concentration of 2,821 pM. The target proteins were each prepared to have a final concentration of 7 nM. As the blocking buffer, a tris buffered saline (also referred to as TBS) containing casein and a nonionic surfactant was used. As shown in Table 3, 6-pattern combinations of BRAF, MEK and ATP were used as reaction compositions. In Table 3, Good indicates that the relevant component is contained, and Poor indicates that the relevant component is not contained.

### (Preparation of antigen-antibody reaction reagent and ICA reaction reagent)

Each mixture of antigen-antibody reaction reagent and the ICA reaction reagent of Pattern 2 contained the above two types of oligonucleotide-modified antibodies, 2 µM allele probe (5'-CGCGCCGAGGAATTGCTCACAGAAAGGA-3') (manufactured by FASMAC, sequence number 3), 4 µM FRET cassette 1 (fluorescent substrate, Alexa 488-BHQ: 5'-X-TTCT-Y-AGCCGGTTTTCCGGCTGAGACCTCGGCGCG-3', X: Alexa 488 + Amino C6, Y: Black hole quencher (BHQ) 1-dT) (manufactured by Japan Bio Services, sequence number 4), 0.108 µM flap endonuclease (FEN)-1, 50 mM Tris-HCl (pH 8.5), and 0 .05% Tween 20. The two types of oligonucleotide-modified antibodies were each prepared to have a final concentration of 1 nM when bound to DNA1, and to have a final concentration of 4 nM when bound to DNA2. The volume of the antigen-antibody reaction reagent used was 2.5 µL, and that of the ICA reaction reagent was 10 µL. The concentrations of the components in the ICA reaction reagent are the final concentrations in the experimental examples.

In Pattern 2, a kinase assay solution was introduced into a tube to undergo a reaction at 30°C for 60 minutes, followed by introducing the antigen-antibody reaction reagent and ICA reaction reagent into the tube. The mixture was reacted at 37°C for 60 minutes, then at 66°C for 60 minutes to 120 minutes, and an immuno-ICA reaction was performed as in Experimental Example 1. The mutant BRAF and the wild-type BRAF were used as proteins of interest.

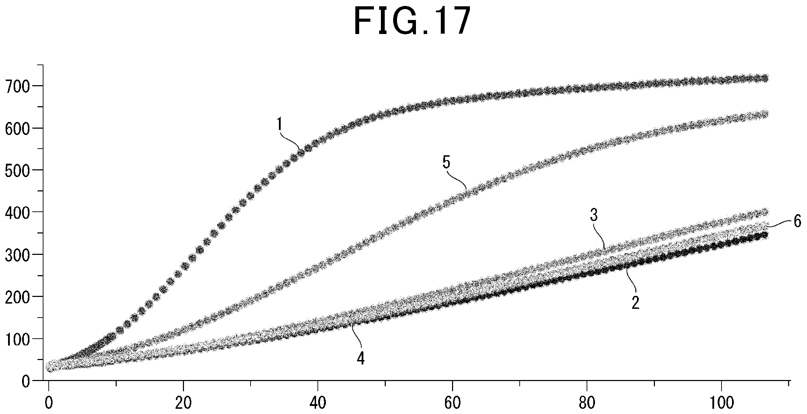

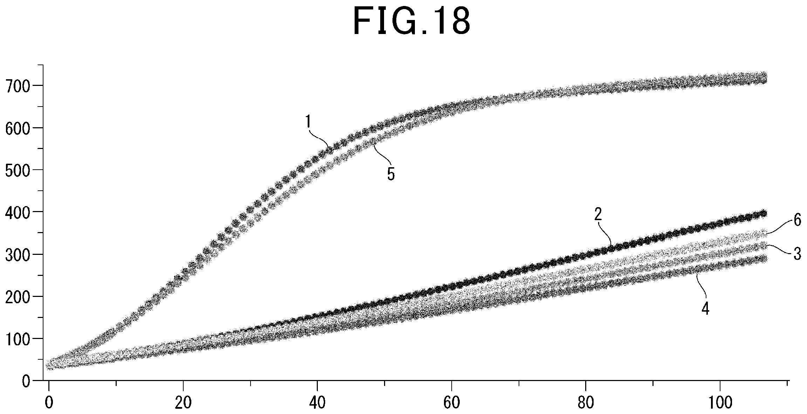

Fig. 17 is a graph showing the results of Pattern 1 of Experimental Example 4. Fig. 18 is a graph showing the results of Pattern 2 of Experimental Example 4. The labels 1 to 6 of Figs. 17 and 18 correspond to Reaction Compositions 1 to 6 of Table 3. Figs. 17 and 18 each show the results of detecting phosphorylation as a result of performing an immuno-ICA reaction for 60 minutes using the mutant BRAF (Mt) or the wild-type BRAF (WT) at a concentration of 2,821 pM as a protein of interest.

Comparing Pattern 1 of Reaction Composition 5 shown in Fig. 17 with Pattern 2 of Reaction Composition 5 shown in Fig. 18, it was clear that Pattern 2, i.e., the case of adding the antibody reaction solution and ICA reaction reagent after the kinase assay, achieved improved reactivity. It was also clear that the ICA reaction could be detected within at least 60 minutes from the start of the reaction. From the results of Pattern 2 of Reaction Composition 3, it was found that no increase in background reaction was observed. The S/N ratios calculated from the results of Patterns 2 of Reaction Compositions 5 and 3 were found to be approximately 4 at maximum.

### [Experimental Example 5]

### (Kinase activity evaluation for proteins of interest (mutant BRAF and wild-type BRAF) in fluidic device 3)

Phosphorylated MEK was detected as in Experimental Example 4, except that an immuno-ICA reaction was performed using the fluidic devices as shown in Fig. 3 to 5. The mutant BRAF and the wild-type BRAF were used as proteins of interest.

A kinase assay and antigen-antibody reaction were performed in tube, and an ICA reaction was performed in a fluidic device. In Pattern 1, each mixture was mixed with the ICA reaction reagent to undergo a kinase assay and antigen-antibody reaction, and then the mixture was introduced into the fluidic device. Following the reaction at 30°C for 60 minutes, phosphorylated MEK was detected as in Experimental Examples 2 and 3. The time of the immuno-ICA reaction was set to be within 60 minutes to 180 minutes. In Pattern 2, a kinase assay solution was reacted at 30°C for 60 minutes, then mixed with the antigen-antibody reaction agent and ICA reaction agent, followed by reaction at 37°C for 60 minutes, and then the mixture was introduced into the fluidic device. Following reaction at 66°C for 60 minutes to 120 minutes, phosphorylated MEK was detected as in Experimental Examples 2 and 3 (Pattern 2).

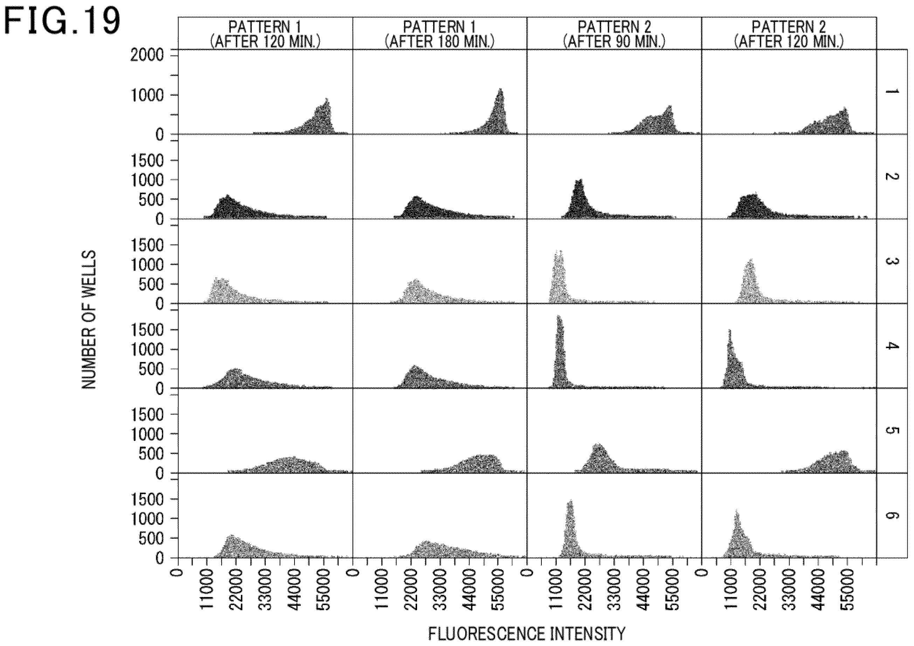

Fig. 19 is a histogram showing measurements of fluorescence intensity over time in immuno-ICA reactions. Fig. 19 shows, from the left, the fluorescence intensity after 120 minutes from the start of the immuno-ICAreaction in Pattern 1, the fluorescence intensity after 180 minutes from the start of the immuno-ICA reaction in Pattern 1, the fluorescence intensity after 90 minutes from the start of the immuno-ICA reaction in Pattern 2, and the fluorescence intensity after 120 minutes from the start of the immuno-ICA reaction in Pattern 2. Numerals 1 to 6 of Fig. 19 correspond to Reaction Compositions 1 to 6 of Table 3.

As can be seen from the results of Fig. 19, it was clear that Pattern 2, i.e., the case of adding the antigen-antibody reaction solution and ICA reaction reagent after the kinase assay, achieved improved reactivity. It is considered that the presence of an antibody during the kinase assay inhibits the phosphorylation reaction.

### [Industrial Applicability]

According to the present invention, there can be provided a technique for evaluating mutant protein activity easily and in a short period of time.

### [Reference Signs List]

- 100, 900: Complex
- 110: Protein of interest
- 120: Target protein
- 131: First single-stranded nucleic acid fragment
- 130: First specific binding substance
- 141: Second single-stranded nucleic acid fragment
- 140: Second specific binding substance
- 150: Double-stranded nucleic acid
- 171: Third single-stranded nucleic acid fragment
- 170: Third specific binding substance
- 200, 500: Fluidic device
- 210: Substrate
- 220: Lid member
- 221: Protrusion
- 222: Inlet port
- 223: Outlet port
- 230: Channel
- 240: Well array
- 241: Well
- 242: Sealed well (microcompartment)
- L210: Reagent solution
- L220: Sealing liquid
- 242R: Signal-detected well
- 510: Wall member
- 810: Flap probe
- 811: First flap site (nucleic acid fragment)
- 821: Second flap site (nucleic acid fragment)
- 820, 820': Nucleic acid fragment
- F: Fluorescent substance
- Q: Quenching substance

## Claims

1. A method of evaluating whether a protein of interest binds to a target protein, comprising
a step (a) of bringing the protein of interest, the target protein, a first specific binding substance which is for the protein of interest and is labeled with a first single-stranded nucleic acid fragment, and a second specific binding substance which is for the target protein and is labeled with a second single-stranded nucleic acid fragment, into contact with each other in a container, and forming as a result a complex comprising the protein of interest, the target protein, the first specific binding substance, and the second specific binding substance when the protein of interest binds to the target protein, with a double-stranded nucleic acid being formed by hybridization of at least part of the first single-stranded nucleic acid fragment with at least part of the second single-stranded nucleic acid fragment; and
a step (b) of detecting formation of the double-stranded nucleic acid, wherein
detection of formation of the double-stranded nucleic acid indicates binding of the protein of interest to the target protein.

2. The method according to claim 1, wherein the step (a) comprises
a step (a1) of synthesizing the protein of interest in the container using a cell-free protein synthesis system; and
a step (a2) of bringing the protein of interest, the target protein, the first specific binding substance, and the second specific binding substance, into contact with each other in the container, and forming as a result a complex comprising the protein of interest, the target protein, the first specific binding substance, and the second specific binding substance when the protein of interest binds to the target protein, with a double-stranded nucleic acid being formed by hybridization of at least part of the first single-stranded nucleic acid fragment with at least part of the second single-stranded nucleic acid fragment.

3. The method according to claim 2, wherein
in the step (a2), adenosine triphosphate (ATP) is additionally brought into contact with the protein of interest, the target protein, the first specific binding substance, and the second specific binding substance.

4. The method according to any one of claims 1 to 3, wherein
the method does not include any cleaning process.

5. The method according to any one of claims 1 to 3, wherein the container comprises wells, and each of the wells has a volume of 10 fL to 100 pL.

6. The method according to any of claims 1 to 3, wherein the first single-stranded nucleic acid fragment and the second single-stranded nucleic acid fragment each have a length of 10 to 200 bases.

7. The method according to any one of claims 1 to 3, wherein the step of detecting formation of the double-stranded nucleic acid is performed through an invasive cleavage assay.

8. A complex comprising a protein of interest, a target protein, a first specific binding substance which is for the protein of interest and is labeled with a first single-stranded nucleic acid fragment, and a second specific binding substance which is for the target protein and is labeled with a second single-stranded nucleic acid fragment, with a double-stranded nucleic acid being formed by hybridization of at least part of the first single-stranded nucleic acid fragment with at least part of the second single-stranded nucleic acid fragment.

9. A kit for evaluating whether a protein of interest binds to a target protein, comprising
a well array having a plurality of wells;
a first specific binding substance which is for the protein of interest and is labeled with a first single-stranded nucleic acid fragment; and
a second specific binding substance which is for the target protein and is labeled with a second single-stranded nucleic acid fragment.

10. The kit according to claim 9, further comprising ATP.

11. The kit according to claim 9 or 10, further comprising a sealing liquid for sealing openings of the wells.

12. The kit according to claim 9 or 10, further comprising a reagent for detecting a double-stranded nucleic acid formed by hybridization of at least part of the first single-stranded nucleic acid fragment with at least part of the second single-stranded nucleic acid fragment.

13. A method of evaluating whether a protein of interest phosphorylates a target protein, comprising
a step (a') of bringing the protein of interest, the target protein, the second specific binding substance which is for the target protein and is labeled with a second single-stranded nucleic acid fragment, a third specific binding substance which is for the target protein undergone phosphorylation and is labeled with a third single-stranded nucleic acid fragment, and ATP, into contact with each other in a container, and forming as a result a complex comprising the target protein, the second specific binding substance, and the third specific binding substance when the target protein is phosphorylated, with a double-stranded nucleic acid being formed by hybridization of at least part of the second single-stranded nucleic acid fragment with at least part of the third single-stranded nucleic acid fragment; and
a step (b) of detecting formation of the double-stranded nucleic acid, wherein
detection of formation of the double-stranded nucleic acid indicates phosphorylation of the target protein.
